# EUROPEAN PATENT APPLICATION

(11) **EP 4 778 568 A1**
(43) Date of publication of application: **22.07.2026**
(21) Application number: 24872199.5
(22) Date of filing: 24.09.2024
(51) Int. Cl.: A61N 5/06, A61M 25/10

(54) **BALLOON, LIGHT IRRADIATION DEVICE, AND TREATMENT METHOD**

(30) Priority: 28.09.2023 JP 2023168773
(71) Applicant: Terumo Kabushiki Kaisha, Tokyo 151-0072 (JP)
(72) Inventor: TOHMA Daiki, Ashigarakami-gun, Kanagawa 259-0151 (JP); SUEHARA Satoru, Ashigarakami-gun, Kanagawa 259-0151 (JP)
(74) Representative: Prüfer & Partner mbB Patentanwälte · Rechtsanwälte
(86) International application number: PCT/JP2024/033972
(87) International publication number: WO 2025/070409

(57) **Abstract**

A balloon according to the present disclosure is a balloon that can be inserted into a living body, the balloon including an expansion portion that defines a central axis extending along an insertion direction from a proximal side to a distal side, the expansion portion including a transmissive layer capable of transmitting light emitted inside to an outside, and a light shielding layer that covers the transmissive layer and has a transmittance of the light lower than that of the transmissive layer, the expansion portion including a window region in which the transmissive layer is not covered with the light shielding layer and a light shielding region in which the transmissive layer is covered with the light shielding layer, and the window region of the expansion portion in an expanded state including a first window portion, and a second window portion arranged spaced apart from the first window portion on the proximal side with respect to the first window portion and more inclined with respect to the central axis than the first window portion.

## Description

### Technical Field

The present disclosure relates to a balloon, a light irradiation device, and a therapeutic method.

### Background Art

A light irradiation device to be used to irradiate an abnormal tissue with therapeutic light is known. Patent Literature 1 discloses this type of light irradiation device. In the light irradiation device described in Patent Literature 1, efficiency and uniformity of light distribution in a treatment region can be improved by including a balloon coated with a reflective material.

### Citation List

### Patent Literature

Patent Literature 1: JP 2005-46640 A

### Summary of Invention

### Technical Problem

A light irradiation device intended to irradiate a target site such as an abnormal tissue with therapeutic light is required to obtain a stable light treatment effect by clarifying a light irradiation region and maintaining light irradiation intensity in the light irradiation region more uniformly. Patent Literature 1 describes a balloon catheter device as a light irradiation device that more uniformly and more efficiently distributes light over an entire light irradiation region. However, with the balloon of the light irradiation device described in Patent Literature 1, an end portion on a front side (proximal side) is coated with the reflective material to define the light irradiation region, and thus, it is difficult for an operator to visually recognize the light irradiation region of the light irradiation device from the front side (proximal side) when irradiating a target site such as an abnormal tissue from the inside of a living body, and it may be difficult to execute reliable treatment.

An object of the present disclosure is to provide a balloon that allows a light irradiation region to be visually recognized from a proximal side in a living body, a light irradiation device including the balloon, and a therapeutic method using the balloon.

### Solution to Problem

A balloon as a first aspect of the present disclosure is
(1)
   a balloon insertable into a living body,
   the balloon including an expansion portion that defines a central axis extending along an insertion direction from a proximal side to a distal side,
   the expansion portion including:
      a transmissive layer capable of transmitting light emitted inside to an outside; and
      a light shielding layer covering the transmissive layer and having a transmittance of the light lower than a transmittance of the transmissive layer,
      the expansion portion including:
         a window region in which the transmissive layer is not covered with the light shielding layer; and
         a light shielding region in which the transmissive layer is covered with the light shielding layer, and
         the window region of the expansion portion in an expanded state including:
            a first window portion; and
            a second window portion arranged spaced apart on the proximal side with respect to the first window portion and more inclined with respect to the central axis than the first window portion.

A balloon as one embodiment of the present disclosure is
(2)
the balloon according to (1), in which the first window portion is substantially parallel or parallel to the central axis.

A balloon as one embodiment of the present disclosure is
(3)
the balloon according to (1) or (2), in which the second window portion is disposed over an entire region in a circumferential direction around the central axis.

A balloon as one embodiment of the present disclosure is
(4)
the balloon according to any one of (1) to (3), in which in the expansion portion, a distal side of the first window portion is comprised only of the light shielding region.

A balloon as one embodiment of the present disclosure is
(5)
the balloon according to any one of (1) to (4), in which
the expansion portion in the expanded state includes:
   a cylindrical expansion main body portion surrounding the central axis; and
   an expansion proximal portion continuous with a proximal side of the expansion main body portion and inclined toward the proximal side to approach the central axis,
   the first window portion is arranged in the expansion main body portion, and
   the second window portion is arranged in the expansion proximal portion.

A balloon as one embodiment of the present disclosure is
(6)
the balloon according to (5), in which the expansion proximal portion protrudes in a hemispherical shape from the expansion main body portion to the proximal side.

A balloon as one embodiment of the present disclosure is
(7)
the balloon according to any one of (1) to (6), in which the first window portion is disposed over a limited angular range not over an entire circumference in a circumferential direction around the central axis of the expansion portion.

A balloon as one embodiment of the present disclosure is
(8)
the balloon according to (7), in which at least a portion of the second window portion is arranged at a position different from the first window portion in the circumferential direction.

A light irradiation device as a second aspect of the present disclosure is
(9)
a light irradiation device including:
   the balloon according to any one of (1) to (8); and
   a tubular body that internally defines an accommodation space accommodating or capable of accommodating a light irradiation unit that emits the light,
   in which the balloon covers a circumference in a radial direction of the tubular body.

A therapeutic method as a third aspect of the present disclosure is
(10)
a therapeutic method using the balloon according to any one of (1) to (8),
the therapeutic method including
a balloon position adjustment step of adjusting a position of the first window portion while visually recognizing the first window portion through the second window portion.

A therapeutic method as one embodiment of the present disclosure is
(11)
the therapeutic method according to (10), in which in the balloon position adjustment step, the position of the first window portion is adjusted by moving the balloon in a direction parallel to the central axis.

(12) The therapeutic method according to (10) or (11), in which in the balloon position adjustment step, the position of the first window portion is adjusted by rotating the balloon in a circumferential direction around the central axis.

A therapeutic method as a fourth aspect of the present disclosure is
(13)
a therapeutic method using the balloon according to any one of (1) to (8), in which
the therapeutic method includes:
   an imaging device insertion step of inserting an imaging device into a living body;
   a balloon insertion step of inserting the balloon into the living body through a through passage of the imaging device or in parallel with an outer surface of the imaging device;
   an expansion step of expanding the balloon in the living body;
   a lens position adjustment step of bringing a lens at a distal end of the imaging device close to the second window portion of the balloon;
   a balloon position adjustment step of adjusting a position of the first window portion so that a scheduled light irradiation region in the living body and the position of the first window portion are aligned while visually recognizing the first window portion through the second window portion; and
   a light irradiation step of irradiating an inside of the living body with the light in a state in which the scheduled light irradiation region and the position of the first window portion are aligned.

A therapeutic method as a fifth aspect of the present disclosure is
(14)
a therapeutic method using the balloon according to any one of (1) to (8), in which
the therapeutic method includes:
   an imaging device insertion step of inserting an imaging device into a living body;
   a balloon insertion step of inserting the balloon into the living body through a through passage of the imaging device or in parallel with an outer surface of the imaging device;
   a first expansion step of expanding the balloon in the living body while adjusting the balloon to a first expansion amount;
   a lens position adjustment step of bringing a lens at a distal end of the imaging device close to the second window portion of the balloon;
   a balloon position adjustment step of adjusting a position of the first window portion of the balloon adjusted to the first expansion amount so that a scheduled light irradiation region in the living body and the position of the first window portion are aligned while visually recognizing the first window portion through the second window portion;
   a second expansion step of expanding the balloon in the living body while adjusting the balloon to a second expansion amount larger than the first expansion amount after the balloon position adjustment step; and
   a light irradiation step of irradiating an inside of the living body with the light in a state in which the scheduled light irradiation region and the position of the first window portion are aligned after the second expansion step.

### Advantageous Effects of Invention

According to the present disclosure, it is possible to provide a balloon that allows a light irradiation region to be visually recognized from a proximal side in a living body, a light irradiation device including the balloon, and a therapeutic method using the balloon.

### Brief Description of Drawings

[Fig. 1] Fig. 1 is a view illustrating an endoscope system including a light irradiation device as an embodiment of the present disclosure including a balloon as an embodiment of the present disclosure.
[Fig. 2A] Fig. 2A is a view illustrating an example of a therapeutic method using the balloon illustrated in Fig. 1, and is a view illustrating a state in which an endoscope as an imaging device is inserted into a hollow organ.
[Fig. 2B] Fig. 2B is a view illustrating a state in which the light irradiation device including the balloon illustrated in Fig. 1 is inserted into the hollow organ from the state of Fig. 2A.
[Fig. 2C] Fig. 2C is a view illustrating a state in which an expansion portion of the balloon is expanded from the state of Fig. 2B.
[Fig. 2D] Fig. 2D is a view illustrating a state in which the expansion portion of the balloon is further expanded and an optical fiber as a light irradiation unit is caused to emit light from the state of Fig. 2C.
[Fig. 3A] Fig. 3A is a perspective view illustrating an expanded state of the balloon illustrated in Fig. 1.
[Fig. 3B] Fig. 3B is a plan view of the balloon illustrated in Fig. 3A as viewed from a proximal side in an axial direction.
[Fig. 4] Fig. 4 is a cross-sectional view taken along a line a-a in Fig. 3B.
[Fig. 5A] Fig. 5A is a perspective view illustrating an expanded state of a balloon as a modification of the balloon illustrated in Fig. 1.
[Fig. 5B] Fig. 5B is a plan view of the balloon illustrated in Fig. 5A as viewed from a proximal side in an axial direction.
[Fig. 6A] Fig. 6A is a perspective view illustrating an expanded state of a balloon as a modification of the balloon illustrated in Fig. 1.
[Fig. 6B] Fig. 6B is a plan view of the balloon illustrated in Fig. 6A as viewed from a proximal side in an axial direction.
[Fig. 7] Fig. 7 is a flowchart indicating an example of a therapeutic method using the balloon illustrated in Fig. 1.

### Description of Embodiments

Hereinafter, embodiments of a balloon, a light irradiation device, and a therapeutic method according to the present disclosure will be described with reference to the drawings. In the drawings, the same components are denoted by the same reference numerals.

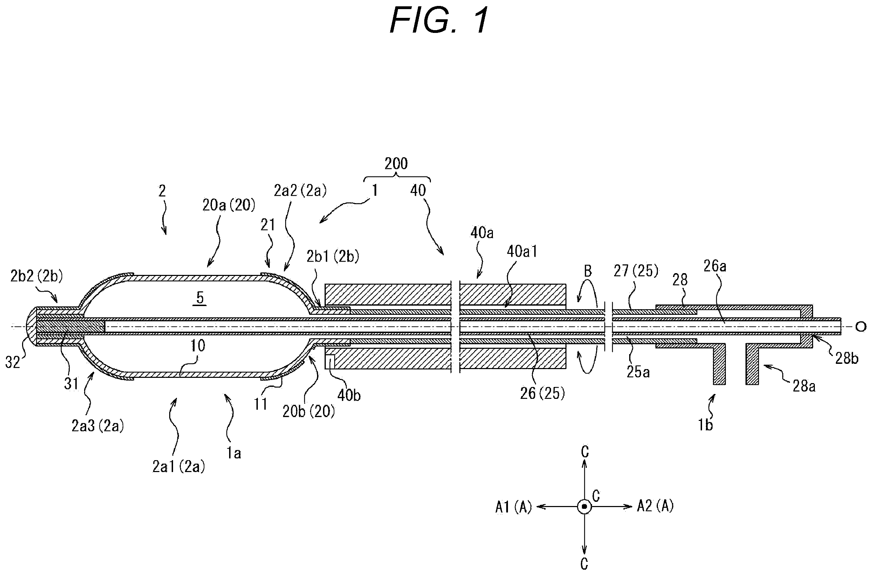

Fig. 1 is a view illustrating an endoscope system 200 including a light irradiation device 1 as an embodiment of the present disclosure including a balloon 2 as an embodiment of the present disclosure. The endoscope system 200 includes the light irradiation device 1 and an endoscope 40. Fig. 1 illustrates a state in which the light irradiation device 1 is inserted through a through passage 40a1 of the endoscope 40. The light irradiation device 1 can be used, for example, for treatment by photodynamic therapy (PDT) or treatment by photoimmunotherapy (PIT). In the present embodiment, the light irradiation device 1 is used, for example, in photoimmunotherapy taught in Japanese Patent No. 6127045 in which a drug adsorbed to a target cell is irradiated with light to destroy the target cell. The target cell is a tumor cell such as a cancer cell or a cell of a precancerous lesion. In this therapeutic method, a photosensitizer in which an antibody that specifically binds to only a specific antigen on the surface of a tumor cell and a photosensitizer paired with the antibody are adsorbed is used as a drug. The antibody is not particularly limited, and examples thereof include panitumumab, trastuzumab, HuJ591, pertuzumab, lapatinib, palbociclib, olaparib, and the like. The photosensitizer is, for example, but not limited to, hydrophilic phthalocyanine, which is a substance reactive to near infrared rays having a wavelength of about 700 nm (IR700). When IR700 receives near infrared rays having a wavelength of about in a range of 660 to 740 nm, a ligand of a functional group that ensures water solubility is broken, and a structural change from water solubility to hydrophobicity occurs. The membrane protein is extracted by this structural change, a hole is formed in the cell membrane, and water enters the cell, whereby the tumor cell can be ruptured and destroyed. In addition, the IR700 is excited by receiving near infrared rays and emits fluorescence having a wavelength different from the excitation wavelength. For example, when the IR700 is excited by receiving near infrared rays having a wavelength around 690 nm, the IR700 emits fluorescence having a wavelength around 700 nm. The IR700 undergoes a structural change while emitting fluorescence by photoreaction, and does not emit fluorescence when it destroys tumor cells and serves as a drug. Fig. 1 illustrates an example in which the light irradiation device 1 is used by being inserted through the through passage 40a1 of the endoscope 40, but the present disclosure is not limited to this use example. The light irradiation device 1 may be used together with, for example, an imaging device different from the endoscope 40 capable of imaging the balloon 2 described later from a proximal side. In addition, the light irradiation device 1 may be inserted into a target tissue such as a hollow organ 80 along an outer surface of the endoscope 40 without being inserted through the through passage 40a1. In addition, the light irradiation device 1 may be used alone without using an imaging device such as the endoscope 40.

In the present disclosure, a longitudinal direction parallel to a central axis O of the light irradiation device 1 is referred to as an "axial direction A of the light irradiation device 1" or simply an "axial direction A". In addition, a direction around the central axis O of the light irradiation device 1 will be referred to as a "circumferential direction B of the light irradiation device 1" or simply a "circumferential direction B". Furthermore, a radial direction of a circle around the central axis O of the light irradiation device 1 will be referred to as a "radial direction C of the light irradiation device 1" or simply a "radial direction C".

As illustrated in Fig. 1, the light irradiation device 1 includes an insertion portion 1a and an operation portion 1b. The insertion portion 1a is located at one end portion of the light irradiation device 1 and can be inserted into an organ having a hollow structure of a living body such as a digestive tract (such as esophagus, stomach, small intestine, and large intestine), a urinary tract, or a blood vessel. The organ having the hollow structure includes, for example, an external auditory canal, an auditory tube, the hollow organ 80, and the like. Examples of the hollow organ 80 include a digestive tract (such as esophagus, stomach, small intestine, large intestine, and biliary tract), a urinary tract (such as ureter and urethra), a blood vessel, a nasal cavity, a trachea, a vagina, a cervical duct, a uterine cavity, a fallopian tube, and the like. The operation portion 1b is located outside the living body in a state where the insertion portion 1a is inserted into the living body, and is operated by an operator. In the present disclosure, a front side to be operated outside the living body in the axial direction A of the light irradiation device 1 will be referred to as a "proximal side". In addition, a side of the light irradiation device 1 to be inserted into the living body in the axial direction A will be referred to as a "distal side". Hereinafter, a direction from the proximal side to the distal side may be referred to as an "insertion direction A1". In addition, a direction from the distal side toward the proximal side may be referred to as a "removal direction A2".

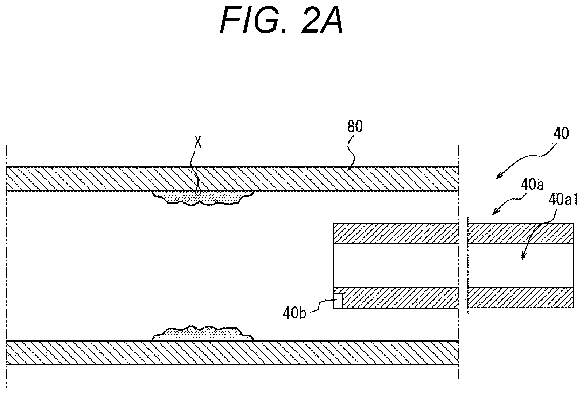

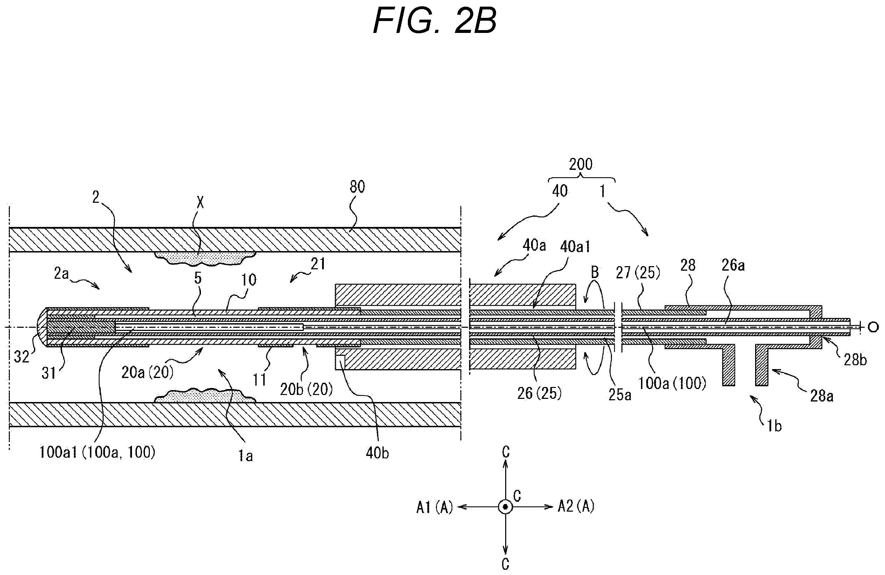

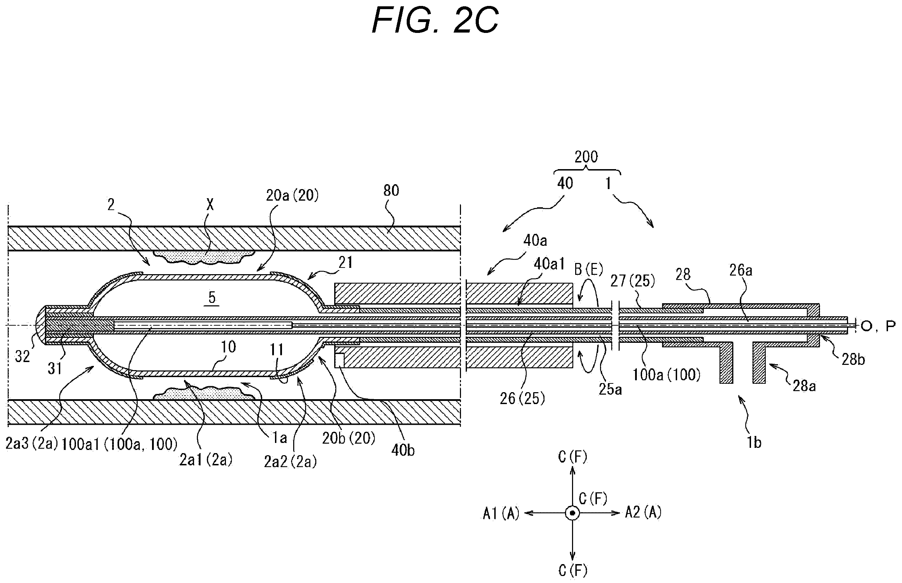

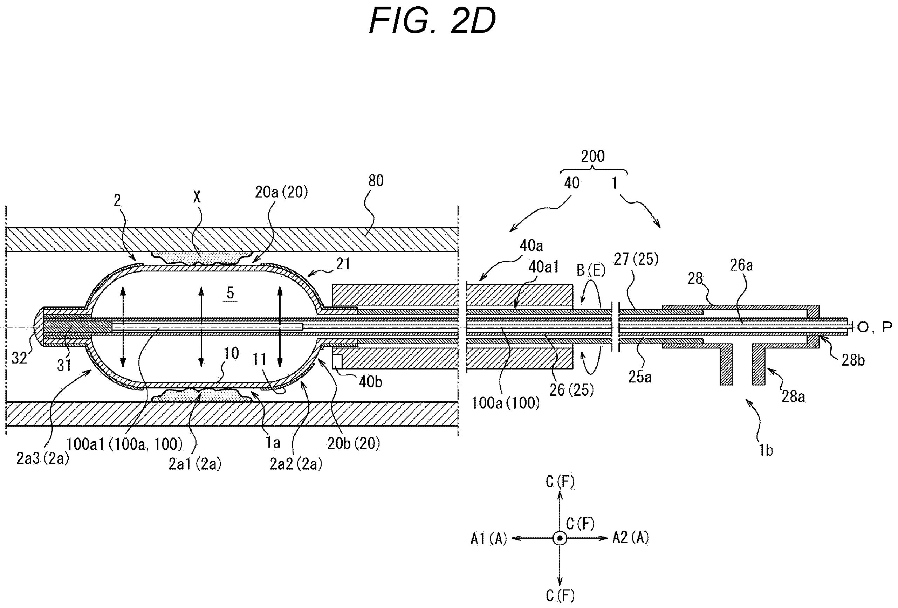

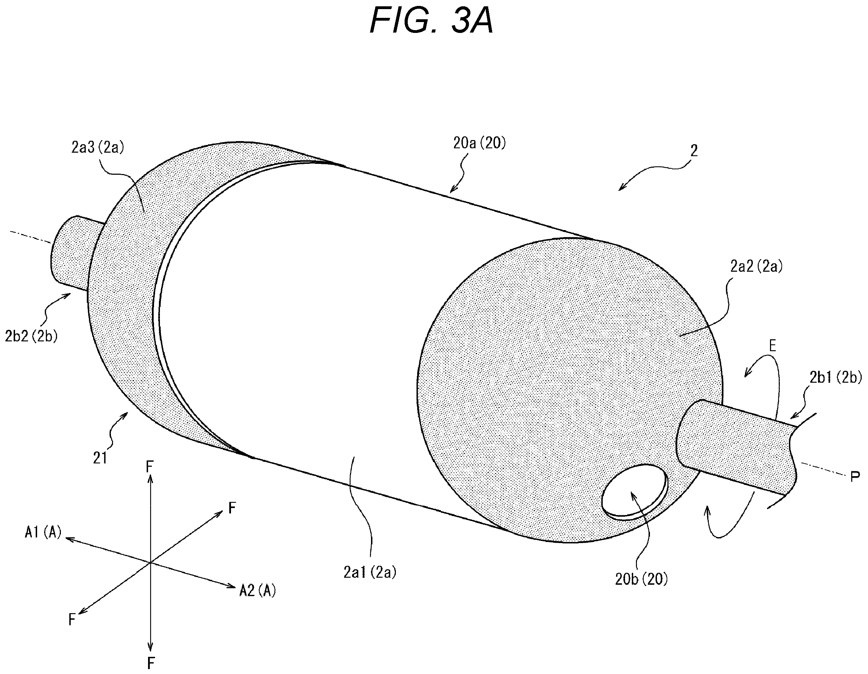

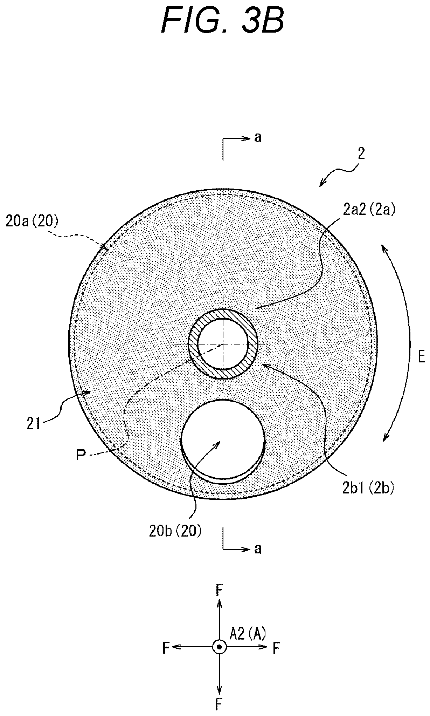

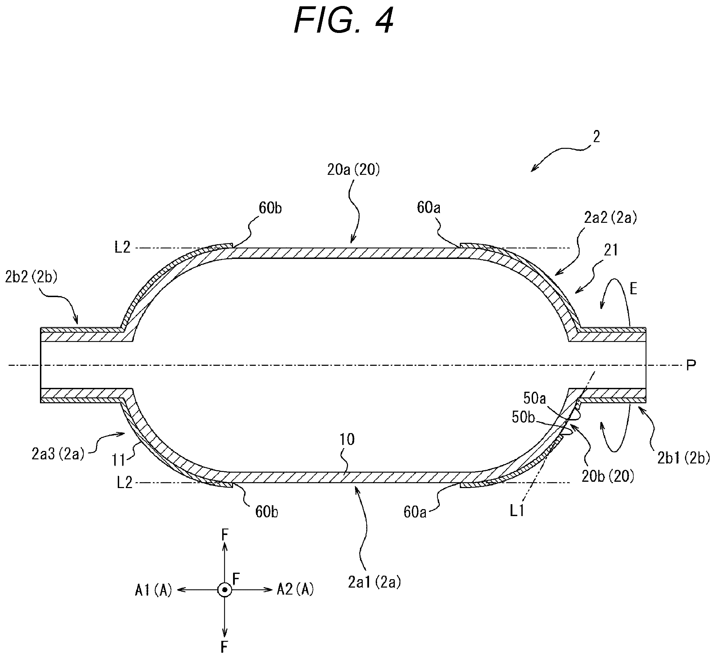

Figs. 2A to 2D are views illustrating an example of a therapeutic method using the light irradiation device 1. Fig. 2A is a view illustrating a state in which the endoscope 40 is inserted into the hollow organ 80. Fig. 2B is a view illustrating a state in which the light irradiation device 1 including the balloon 2 is inserted into the hollow organ 80 from the state of Fig. 2A. Fig. 2C is a view illustrating a state in which an expansion portion 2a of the balloon 2 is expanded from the state of Fig. 2B. Fig. 2D is a view illustrating a state in which the expansion portion 2a of the balloon 2 is further expanded and an optical fiber 100a as the light irradiation unit 100 is caused to emit light from the state of Fig. 2C. The optical fiber 100a may be formed with, for example, a side emission fiber from which light is emitted all around the outside in the radial direction C. Fig. 3A is a perspective view illustrating an expanded state of the balloon 2 of the present embodiment. Fig. 3B is a plan view of the balloon 2 illustrated in Fig. 3A as viewed from the proximal side in the axial direction A. Fig. 4 is a cross-sectional view taken along a line a-a in Fig. 3B. Fig. 4 is a view illustrating an example of a cross section including a central axis P of the expansion portion 2a of the balloon 2 in the expanded state and parallel to the central axis P. Furthermore, the cross section illustrated in Fig. 4 is a cross section passing through a first window portion 20a and a second window portion 20b of the expansion portion 2a of the balloon 2.

As illustrated in Fig. 2B, and the like, the light irradiation device 1 of the present embodiment includes the balloon 2 that can be inserted into the hollow organ 80, a tubular member 25, a hub 28, and a distal end member 32. The distal end member 32 forms a distal end (hereinafter, referred to as a "distal end") of the light irradiation device 1. A first tubular body 26 of the tubular member 25 is connected to the proximal side of the distal end member 32 and extends to the proximal end (hereinafter, referred to as a "proximal end") of the light irradiation device 1. The balloon 2 is attached to an end portion on a distal side (hereinafter, referred to as a "distal end portion") of the tubular member 25. The hub 28 is attached to the tubular member 25 and includes a port portion 28a to which a fluid supply tool can be connected. The insertion portion 1a of the present embodiment includes the balloon 2 and a portion on the distal side of the tubular member 25. In addition, the operating portion 1b of the present embodiment includes the hub 28 and a portion on the proximal side of the tubular member 25. Here, the central axis O of the light irradiation device 1 of the present embodiment coincides with the central axes of the first tubular body 26 and a second tubular body 27 of the tubular member 25. In other words, the axial direction A of the light irradiation device 1 of the present embodiment is the same as the axial direction of the first tubular body 26 and the second tubular body 27 of the tubular member 25 parallel to the central axes of the first tubular body 26 and the second tubular body 27 of the tubular member 25. In addition, the circumferential direction B of the light irradiation device 1 of the present embodiment is the same as the circumferential direction of the first tubular body 26 and the second tubular body 27 of the tubular member 25 around the central axes of the first tubular body 26 and the second tubular body 27 of the tubular member 25. Furthermore, the radial direction C of the light irradiation device 1 of the present embodiment is the same as the radial direction of the first tubular body 26 and the second tubular body 27 of the tubular member 25, which is the radial direction of a circle around the central axis of the first tubular body 26 and the second tubular body 27 of the tubular member 25.

The distal end member 32 of the present embodiment is a disk plate portion arranged substantially coaxially with the tubular member 25. A distal surface of the disk plate portion as the distal end member 32 of the present embodiment is configured by a curved convex surface protruding to the distal side. By forming the distal surface of the disk plate portion into a curved convex surface, the inside of the living body is hardly damaged when the insertion portion 1a is inserted into the living body such as the hollow organ 80. However, a shape of the distal end member 32 is not limited thereto. In addition, the light irradiation device 1 does not have to include the distal end member 32 as long as the distal end is closed.

The tubular member 25 internally defines an accommodation space 26a capable of accommodating the light irradiation unit 100 and an inflation lumen 25a capable of supplying a fluid such as air into the balloon 2. More specifically, the tubular member 25 of the present embodiment includes the first tubular body 26 and the second tubular body 27. The first tubular body 26 and the second tubular body 27 are arranged concentrically. The second tubular body 27 is arranged so as to surround the outside of the first tubular body 26 in the radial direction C. The first tubular body 26 protrudes to the distal side of the second tubular body 27. The first tubular body 26 protrudes to the proximal side of the second tubular body 27. Furthermore, the first tubular body 26 of the present embodiment protrudes to the proximal side of the hub 28 connected to the proximal side of the second tubular body 27. However, a proximal end of the first tubular body 26 may be terminated in the hub 28 as long as the optical fiber 100a as the light irradiation unit 100 described later can be inserted. The accommodation space 26a is defined in the first tubular body 26. A distal end of the accommodation space 26a is closed. More specifically, a closing member 31 is arranged inside the distal end portion of the first tubular body 26 of the present embodiment. The distal end of the accommodation space 26a is closed by the closing member 31. However, the distal end of the accommodation space 26a does not have to be closed by the closing member 31. In this case, for example, a diameter of the accommodation space 26a may be reduced to a diameter in which a light emission unit 100a1 cannot be partially inserted so that the light emission unit 100a1 of the optical fiber 100a as the light irradiation unit 100 can be arranged at an appropriate position in the balloon 2. In a case where the distal end member 32 and the closing member 31 are not included, it is possible to prevent the optical fiber 100a as the light irradiation unit 100 from being contaminated with a body fluid such as blood by flowing physiological saline into the living body before the optical fiber 100a as the light irradiation unit 100 is inserted into the accommodation space 26a. The inflation lumen 25a is defined between an inner surface of the second tubular body 27 and an outer surface of the first tubular body 26.

The light irradiation unit 100 can emit light outward in the radial direction C. The light irradiation unit 100 of the present embodiment is the optical fiber 100a. The optical fiber 100a includes the light emission unit 100a1 that emits light outward in the radial direction C at the distal end (see Fig. 2B, and the like). The first tubular body 26 can transmit the light emitted inside to an outside. More specifically, the first tubular body 26 can transmit the light emitted from the light irradiation unit 100 accommodated in the accommodation space 26a outward in the radial direction C of the first tubular body 26. The first tubular body 26 is formed with a light transmissive material capable of transmitting light having a wavelength that can be visually recognized by the endoscope 40 in addition to the therapeutic light emitted from the light irradiation unit 100, and may be formed with, for example, a transparent resin material. For example, an opening through which light can pass may be formed in the first tubular body 26.

The light irradiation device 1 of the present embodiment does not include the light irradiation unit 100. In other words, the light irradiation device 1 of the present embodiment is used together with the optical fiber 100a as the light irradiation unit 100 separately accommodated in the accommodation space 26a of the first tubular body 26. However, the light irradiation device 1 may include the light irradiation unit 100. In this case, the light irradiation unit 100 may be accommodated in the accommodation space 26a and integrated with the light irradiation device 1.

The optical fiber 100a as the light irradiation unit 100 accommodated in the accommodation space 26a of the first tubular body 26 is inserted into the accommodation space 26a until the light emission unit 100a1 reaches the position in the balloon 2 in the axial direction A. In the present embodiment, the optical fiber 100a is inserted into the accommodation space 26a until the light emission unit 100a1 disposed on the distal side of the optical fiber 100a abuts on the closing member 31. In the present embodiment, the light emission unit 100a1 of the optical fiber 100a can be reliably arranged at a predetermined position in the balloon 2 by inserting the light emission unit 100a1 to a position where the light emission unit 100a1 abuts on the closing member 31.

The light irradiation device 1 does not have to include the first tubular body 26. In this case, the balloon 2 may internally define an accommodation space accommodating or capable of accommodating the light irradiation unit 100. However, the light irradiation device 1 includes the first tubular body 26 that defines the accommodation space 26a, and thus, it is easy to stabilize a position of the light irradiation unit 100 in the axial direction A and the radial direction C.

A material for forming the first tubular body 26 and the second tubular body 27 of the tubular member 25 is not particularly limited, but may be, for example, a resin material. Examples of the resin material as the material for forming the first tubular body 26 and the second tubular body 27 include polyolefins such as polyethylene, polypropylene, and an ethylene-propylene copolymer.; Ethylene-vinyl acetate copolymer (EVA); Polyvinyl chloride; Polyvinylidene chloride; Polystyrene; Polyamide; Polyamide-based elastomer; Polyimide; Polyamideimide; Polycarbonate; Poly- (4-methylpentene -1); ionomer; Acrylic resin; Polymethyl methacrylate; Acrylonitrile-butadiene-styrene copolymer (ABS resin); Acrylonitrile-styrene copolymer (AS resin); Butadiene-styrene copolymer; Polyesters such as polyethylene terephthalate (PET), polybutylene terephthalate (PBT), and polycyclohexane terephthalate (PCT); Polyether; Polyetherketone (PEK); Polyetheretherketone (PEEK); Polyetherimide; Polyacetal (POM); Polyphenylene oxide; Modified polyphenylene oxide; Polysulfone; Polyether sulfone; Polyphenylene sulfide; Polyarylate; Aromatic polyester (liquid crystal polymer); Polytetrafluoroethylene, polyvinylidene fluoride, and other fluorine-based resins; and the like. In addition, a blend containing one or more of these may be used. In addition, the second tubular body 27 may be a metal such as stainless steel, iron, a nickel-titanium alloy, or aluminum.

The balloon 2 of the present embodiment covers a circumference of the first tubular body 26 of the tubular member 25 in the radial direction C. More specifically, the balloon 2 of the present embodiment is an endless membrane covering an entire region in the circumferential direction B of the first tubular body 26 on the outer side in the radial direction C of the first tubular body 26. As illustrated in Fig. 1, the balloon 2 of the present embodiment includes the expansion portion 2a that can be expanded and deflated and a protrusion 2b. The protrusion 2b includes a distal protrusion 2b2 connected to the distal end of the expansion portion 2a and protruding to the distal side, and a proximal protrusion 2b1 connected to the proximal end of the expansion portion 2a and protruding to the proximal side. The distal protrusion 2b2 is bonded to the distal end portion of the first tubular body 26 by adhesion, or the like, over the entire region in the circumferential direction B. In addition, the proximal protrusion 2b1 is bonded to the distal end portion of the second tubular body 27 by adhesion, or the like, over the entire region in the circumferential direction B. In the present embodiment, the light shielding layer 11 to be described later is disposed in the distal protrusion 2b2 and the proximal protrusion 2b1, but the light shielding layer 11 to be described later does not have to be disposed in the proximal protrusion 2b1. Then, the expansion portion 2a is not bonded to the first tubular body 26 and the second tubular body 27 of the tubular member 25 and defines an annular space 5 capable of accommodating a fluid between the expansion portion 2a and the first tubular body 26. An opening on the distal side of the inflation lumen 25a defined between the first tubular body 26 and the second tubular body 27 communicates with the annular space 5. The expansion portion 2a of the balloon 2 expands outward in the radial direction C by supplying fluid to the annular space 5 through the inflation lumen 25a. Conversely, as a result of the fluid being discharged from the annular space 5 through the inflation lumen 25a, the expansion portion 2a of the balloon 2 is deflated inward in the radial direction C. In the expansion portion 2a of the balloon 2, an amount of expansion to the outside in the radial direction C increases as a pressure of the fluid supplied to the annular space 5 increases.

However, the balloon 2 is not limited to the configuration including the expansion portion 2a and the protrusion 2b described above. For example, the balloon 2 may have a configuration including the expansion portion 2a and not including the protrusion 2b. In this case, the expansion portion 2a may be bonded to the distal end portions of the first tubular body 26 and the second tubular body 27 by adhesion, or the like, over the entire region in the circumferential direction B.

As illustrated in Figs. 2C to 4, the expansion portion 2a of the balloon 2 defines the central axis P extending along the insertion direction A1 from the proximal side toward the distal side in the expanded state. The central axis P substantially coincides with the central axis O of the light irradiation device 1. In other words, in the present embodiment, a direction parallel to the central axis P defined by the expansion portion 2a in the expanded state is the same as the axial direction A of the light irradiation device 1. Hereinafter, a direction around the central axis P defined by the expansion portion 2a in the expanded state will be referred to as a "circumferential direction E of the expansion portion 2a" or simply a "circumferential direction E". In addition, a radial direction of a circle around the central axis P defined by the expansion portion 2a in the expanded state will be referred to as a "radial direction F of the expansion portion 2a" or simply as a "radial direction F".

Although details will be described later, the expansion portion 2a of the balloon 2 includes the transmissive layer 10 capable of transmitting light emitted inside to the outside, and the light shielding layer 11 covering the transmissive layer 10 and having a lower light transmittance than the transmissive layer 10. The expansion portion 2a of the balloon 2 includes a window region 20 in which the transmissive layer 10 is not covered with the light shielding layer 11, and a light shielding region 21 in which the transmissive layer 10 is covered with the light shielding layer 11. The window region 20 of the expansion portion 2a includes, in the expanded state, the first window portion 20a and the second window portion 20b that is arranged spaced apart from the first window portion 20a on the proximal side and is more inclined with respect to the central axis P than the first window portion 20a. As a result of the expansion portion 2a of the balloon 2 including the first window portion 20a, in the treatment using the balloon 2, it is possible to irradiate the light irradiation region clarified as the first window portion 20a with light with irradiation intensity maintained more uniformly, and it is possible to efficiently perform light irradiation. In addition, as a result of the expansion portion 2a of the balloon 2 including the second window portion 20b, in the treatment using the balloon 2, the first window portion 20a can be visually recognized through the second window portion 20b using an imaging device such as the endoscope 40. The light irradiation region means a region of the balloon 2 defined as a range to be irradiated with light, and means the entire region of the first window portion 20a in the present embodiment.

As illustrated in Fig. 1, and the like, the hub 28 is attached to the proximal end portion of the second tubular body 27 of the tubular member 25 of the present embodiment. As described above, the hub 28 includes the port portion 28a to which a fluid supply tool can be connected. Liquid or gas can be supplied to the annular space 5 of the balloon 2 through the inflation lumen 25a by the fluid supply tool such as a syringe connected to the port portion 28a. The hub 28 of the present embodiment defines a through hole 28b through which the first tubular body 26 is inserted.

As illustrated in Fig. 1 and the like, the endoscope 40 as an imaging device to be used in the endoscope system 200 of the present embodiment includes a main body portion 40a and a lens 40b. The main body portion 40a is an elongated member extending along the axial direction A. The main body portion 40a defines a through passage 40a1 through which the light irradiation device 1 can be inserted. The through passage 40a1 extends from the proximal end to the distal end of the main body portion 40a and penetrates the main body portion 40a. The light irradiation device 1 inserted into the through passage 40a1 of the endoscope 40 is fixed to the endoscope 40 so that relative movement in the axial direction A with respect to the endoscope 40 is restricted. The lens 40b is located at the distal end of the endoscope 40 and collects light incident from the distal side to create an optical image. In a state where the light irradiation device 1 is inserted through the through passage 40a1 of the endoscope 40, the lens 40b may be arranged adjacent to the proximal side of the second window portion 20b of the balloon 2. By arranging the lens 40b adjacent to the proximal side of the second window portion 20b of the balloon 2, light reflected by a target site X (see Fig. 2A, and the like), or the like, to be irradiated with light can pass through the second window portion 20b via the first window portion 20a and enter the lens 40b. The optical image created by the lens 40b can be displayed in real time on a display unit such as a liquid crystal monitor of an image display device connected to the endoscope 40. Furthermore, a light capable of emitting light having a wavelength different from that of light of the light irradiation unit 100 may be disposed at the distal end portion of the endoscope 40, and the inside of the living body (for example, inside of the hollow organ 80) may be illuminated by the light.

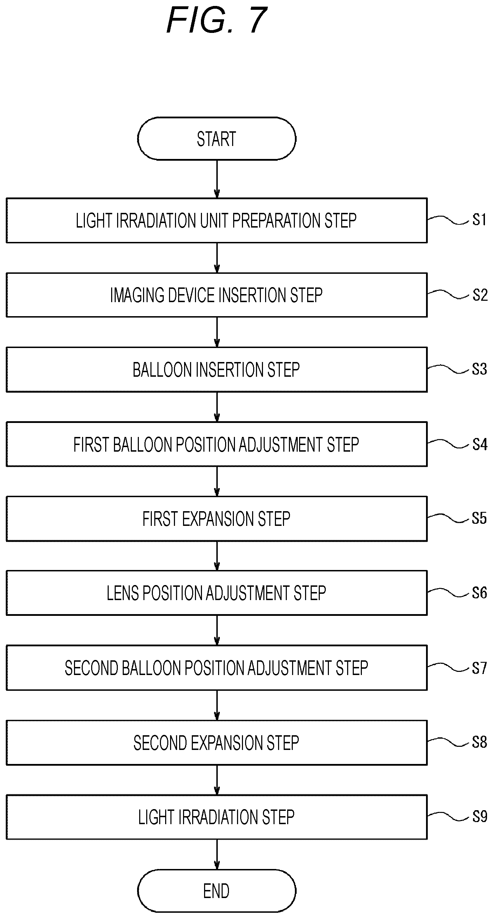

Next, an example of a therapeutic method using the balloon 2 will be described with reference to Figs. 2A to 2D and 7. Fig. 7 is a flowchart indicating an example of the therapeutic method using the balloon 2.

As illustrated in Fig. 7, the therapeutic method using the balloon 2 of the present embodiment includes a light irradiation unit preparation step S1, an imaging device insertion step S2, a balloon insertion step S3, a first balloon position adjustment step S4, a first expansion step S5, a lens position adjustment step S6, a second balloon position adjustment step S7, a second expansion step S8, and a light irradiation step S9. In the following description of the therapeutic method, a photosensitizer as a drug is administered to a patient in advance, and the photosensitizer is adsorbed in a cancer tissue.

In the light irradiation unit preparation step S1, the optical fiber 100a as the light irradiation unit 100 is inserted into the accommodation space 26a of the first tubular body 26 from the proximal side of the light irradiation device 1 including the balloon 2. In this event, the light emission unit 100a1 of the optical fiber 100a is inserted to the position where the balloon 2 is disposed in the axial direction A. The optical fiber 100a as the light irradiation unit 100 may be fixed in a state of being inserted in advance into the accommodation space 26a of the first tubular body 26. In this case, the light irradiation preparation step S1 can be skipped.

In the imaging device insertion step S2, after the light irradiation unit preparation step S1, as illustrated in Fig. 2A, the endoscope 40 as an imaging device is inserted into the hollow organ 80 including the target site X to be treated.

In the balloon insertion step S3, after the imaging device insertion step S2, as illustrated in Fig. 2B, the balloon 2 is inserted into the hollow organ 80 through the through passage 40a1 of the endoscope 40 inserted into the hollow organ 80. More specifically, in the balloon insertion step S3 of the present embodiment, the light irradiation device 1 including the balloon 2 in a state where the optical fiber 100a as the light irradiation unit 100 is inserted is pushed in the insertion direction A1 and inserted into the hollow organ 80 through the through passage 40a1 of the endoscope 40 inserted into the hollow organ 80. In this event, the expansion portion 2a of the balloon 2 is in a deflated state. The balloon insertion step S3 may be performed by inserting the balloon 2 into the hollow organ 80 in parallel along the outer surface of the endoscope 40 without passing through the through passage 40a1.

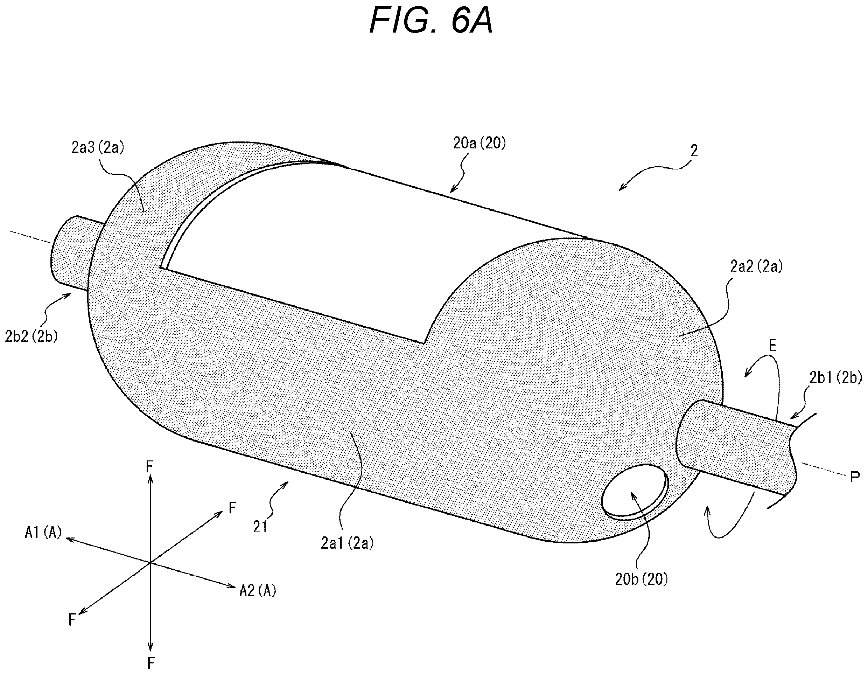

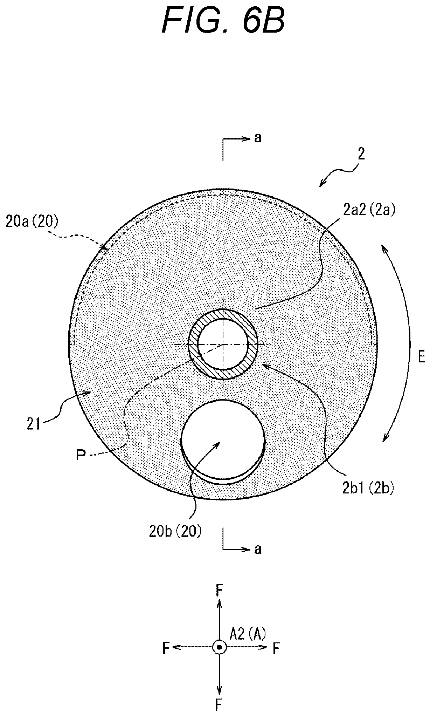

In the first balloon position adjustment step S4, after the balloon insertion step S3, the position of the first window portion 20a of the balloon 2 is adjusted to be aligned with a scheduled light irradiation region including the target site X to be irradiated with light. The first balloon position adjustment step S4 is executed while the expansion portion 2a of the balloon 2 is in the deflated state (see Fig. 2B). Specifically, the position of the first window portion 20a can be adjusted by operating the operation portion 1b of the light irradiation device 1 to move the balloon 2 in a direction parallel to the central axis P (the axial direction A in the present embodiment). As described later, in a case where the first window portion 20a is disposed over a limited angular range not over the entire circumference in the circumferential direction E around the central axis P of the expansion portion 2a (see Figs. 6A and 6B), the position of the first window portion 20a may be adjusted by operating the operation portion 1b of the light irradiation device 1 to move the balloon 2 in the circumferential direction E around the central axis P. One or both of the movement in the direction parallel to the central axis P of the balloon 2 (the axial direction A in the present embodiment) and the movement in the circumferential direction E are executed as necessary. The first balloon position adjustment step S4 is executed while confirming the position of the balloon 2 with the endoscope 40 as an imaging device, for example. The first balloon position adjustment step S4 may be repeatedly executed. The scheduled light irradiation region means a region in the living body determined in advance as a region to be irradiated with light.

In the first expansion step S5, after the first balloon position adjustment step S4, as illustrated in Fig. 2C, the expansion portion 2a of the balloon 2 is expanded, and the deflated state is changed to the expanded state. In this event, the expansion portion 2a of the balloon 2 is expanded while adjusting the expansion amount to a first expansion amount, which is an expansion amount to such an extent that the expansion portion 2a does not contact an inner wall of the hollow organ and the target site X or to such an extent that the expansion portion 2a lightly contacts the inner wall of the hollow organ 80 and the target site X.

In the lens position adjustment step S6, after the first expansion step S5, the lens 40b at the distal end of the endoscope 40 as the imaging device is brought close to the second window portion 20b of the balloon 2. The lens position adjustment step S6 is executed, for example, by relatively moving the endoscope 40 as the imaging device in one or both of the axial direction A and the circumferential directions B and E with respect to the light irradiation device 1. In this event, liquid may be caused to flow between the second window portion 20b and the lens 40b of the endoscope 40 so as to reduce a difference in refractive index between the inside of the balloon 2 and the space between the second window portion 20b and the lens 40b of the endoscope 40. In this manner, by bringing the lens 40b close to the second window portion 20b of the balloon 2, the first window portion 20a can be visually recognized through the second window portion 20b.

In the second balloon position adjustment step S7, after the lens position adjustment step S6, the position of the first window portion 20a of the balloon 2 is adjusted to be aligned with the scheduled light irradiation region including the target site X to be irradiated with light. Specifically, the position of the first window portion 20a can be adjusted by operating the operation portion 1b of the light irradiation device 1 to move the balloon 2 in a direction parallel to the central axis P (the axial direction A in the present embodiment). As described later, in a case where the first window portion 20a is disposed over a limited angular range not over the entire circumference in the circumferential direction E around the central axis P of the expansion portion 2a (see Figs. 6A and 6B), the position of the first window portion 20a may be adjusted by operating the operation portion 1b of the light irradiation device 1 to move the balloon 2 in the circumferential direction E around the central axis P. One or both of the movement in the direction parallel to the central axis P of the balloon 2 (the axial direction A in the present embodiment) and the movement in the circumferential direction E are executed as necessary. In this event, by using the endoscope 40 as the imaging device, the position of the first window portion 20a can be adjusted while the first window portion 20a is visually recognized through the second window portion 20b. In other words, the position of the first window portion 20a can be adjusted while whether or not the scheduled light irradiation region including the target site X is located within the range of the first window portion 20a is confirmed. Thus, the position of the first window portion 20a can be adjusted more accurately as compared with the first balloon position adjustment step S4. Thus, the position of the first window portion 20a can be efficiently adjusted by performing the first balloon position adjustment step S4 and the second balloon position adjustment step S7 step by step. The second balloon position adjustment step S7 may be repeatedly executed. Furthermore, in this event, the inside of the hollow organ 80 can be illuminated as necessary using a light disposed at the distal end portion of the endoscope 40 capable of emitting light having a wavelength different from that of the light of the light irradiation unit 100. As described above, by using light having a wavelength different from that of the light of the light irradiation unit 100, unintended activation of the photosensitizer can be prevented.

In the second expansion step S8, after the second balloon position adjustment step S7, as illustrated in Fig. 2D, the expansion portion 2a of the balloon 2 is expanded while adjusting the expansion portion 2a of the balloon 2 to a second expansion amount larger than the first expansion amount in the first expansion step S5, and the first window portion 20a is brought close to or in close contact with the target site X. By bringing the first window portion 20a close to or in close contact with the target site X, the target site X can be more reliably irradiated with light in the light irradiation step S9 described later. Furthermore, it is possible to more clearly visually recognize the state of the target site X through the first window portion 20a, and it is possible to prevent positional displacement of the balloon 2 during treatment.

In the light irradiation step S9, after the second expansion step S8, as illustrated in Fig. 2D, the optical fiber 100a as the light irradiation unit 100 is caused to emit light, and the scheduled light irradiation region including the target site X is irradiated with light through the first tubular body 26 and the first window portion 20a.

After the series of steps described above is executed, the expansion portion 2a of the balloon 2 is deflated, the light irradiation device 1 is pulled out in the removal direction A2, and the light irradiation device 1 is removed from the living body through the through passage 40a1 of the endoscope 40. However, the insertion portion 1a of the light irradiation device 1 may be removed from the living body together with the endoscope 40.

Next, further details of the balloon 2 will be described.

The expansion portion 2a of the balloon 2 includes the transmissive layer 10 formed with a light transmissive material and the light shielding layer 11 formed with a light reflecting material or a light absorbing material. The light shielding layer 11 covers the transmissive layer 10. More specifically, the light shielding layer 11 of the present embodiment is laminated on the outer surface of the transmissive layer 10 in the radial direction C. The transmissive layer 10 is transparent and can transmit light emitted inside the expansion portion 2a to the outside of the expansion portion 2a. More specifically, the transmissive layer 10 can transmit the light emitted from the optical fiber 100a as the light irradiation unit 100 located inside the expansion portion 2a to the outside of the expansion portion 2a. A wavelength of light from the light irradiation unit 100 may be appropriately selected according to treatment, and may be visible light, for example. On the other hand, the light shielding layer 11 is less likely to transmit the light emitted from the light irradiation unit 100 than the transmissive layer 10. More specifically, in the light shielding layer 11, a transmittance of light emitted from the light irradiation unit 100 is lower than that of the transmissive layer 10.

Examples of the light transmissive material forming the transmissive layer 10 include resin materials such as polyethylene terephthalate, polyurethane, polyamide, polyamide-based elastomer, and silicone rubber. Examples of the light reflecting material forming the light shielding layer 11 include various metal materials such as titanium oxide, barium sulfate, and zinc oxide, and light absorbing pigments such as carbon black. For example, the light shielding layer 11 may be formed on the surface of the transmissive layer 10 by coating. A coating method is not particularly limited, and for example, a dipping method, a spray coating method, a roll coating method, a screen printing method, or the like, may be used. Furthermore, the light shielding layer 11 may be formed on a surface of the transmissive layer 10 by a vapor deposition method. The light shielding layer 11 may be formed on the inner surface of the balloon 2 or formed integrally with the balloon 2.

As illustrated in Figs. 1 and 2B to 3B, the balloon 2 includes the window region 20 and the light shielding region 21. The light shielding region 21 is a region in which the transmissive layer 10 is covered with the light shielding layer 11. On the other hand, the window region 20 is a region where the transmissive layer 10 is not covered with the light shielding layer 11. As a result of the transmissive layer 10 being covered with the light shielding layer 11, the light shielding region 21 hardly transmits the light of the optical fiber 100a as the light irradiation unit 100 compared to the window region 20. More specifically, in the light shielding region 21, the light transmittance of the optical fiber 100a as the light irradiation unit 100 is lower than that of the window region 20.

The window region 20 includes the first window portion 20a and the second window portion 20b which is arranged spaced apart from the first window portion 20a on the proximal side and is more inclined with respect to the central axis P than the first window portion 20a in the expanded state of the expansion portion 2a. As described above, as a result of the second window portion 20b being arranged on the proximal side with respect to the first window portion 20a, the operator can visually recognize the first window portion 20a located on the distal side of the second window portion 20b through the second window portion 20b. In addition, as a result of the second window portion 20b being arranged spaced apart from the first window portion 20a (in other words, as a result of at least a portion of the light shielding region 21 being located between the first window portion 20a and the second window portion 20b), the outer edge of the first window portion 20a can be defined by the light shielding region 21. As a result, when the first window portion 20a is viewed through the second window portion 20b, the range of the first window portion 20a can be clearly visually recognized. In other words, in a case where the second window portion 20b is not arranged spaced apart from the first window portion 20a (that is, in a case where the second window portion 20b and the first window portion 20a are connected to each other), the boundary between the first window portion 20a and the second window portion 20b cannot be visually recognized when the first window portion 20a is viewed through the second window portion 20b. Thus, from the viewpoint of usability, it is preferable that the second window portion 20b is arranged spaced apart from the first window portion 20a.

The light of the optical fiber 100a emitted inside the expansion portion 2a passes through the first tubular body 26 and is emitted toward the outside in the radial direction C of the expansion portion 2a through the first window portion 20a. The light of the optical fiber 100a can also be emitted from the second window portion 20b, but the second window portion 20b is located on the proximal side of the light emission unit 100a1 of the optical fiber 100a, and thus, the intensity of the light emitted from the second window portion 20b is smaller than the intensity of the light emitted from the first window portion 20a. In addition, in the expansion portion 2a, a portion other than a portion where the first window portion 20a and the second window portion 20b are formed is the light shielding region 21. The light shielding region 21 has a lower light transmittance of the optical fiber 100a than the window region 20. Thus, the intensity of the light irradiated to the outside in the radial direction C through the light shielding region 21 is smaller than the intensity of the light irradiated at the first window portion 20a. With such a configuration, it is possible to irradiate the inside of the light irradiation region defined as the first window portion 20a with light with irradiation intensity maintained more uniformly, and efficient light irradiation becomes possible.

In the expansion portion 2a of the present embodiment, the distal side of the first window portion 20a is comprised only of the light shielding region 21. By this means, it is possible to suppress light irradiation to a site other than the scheduled light irradiation region including the target site X (see Figs. 2A to 2D) to be treated in the living body, which is located on the distal side of the first window portion 20a.

The first window portion 20a of the present embodiment is disposed over the entire circumference in the circumferential direction E around the central axis P of the expansion portion 2a. As a result, even in a case where the target site X located on the outer side in the radial direction C of the balloon 2 exists over the entire region in the circumferential direction E of the balloon 2, the entire region in the circumferential direction E of the target site X can be irradiated with light through the first window portion 20a.

A maximum length of the second window portion 20b may be, for example, 1.8 cm or less in plan view as viewed from the axial direction A. Further, the maximum length of the second window portion 20b may be 1.5 cm or less or 1.2 cm or less in plan view as viewed from the axial direction A. With this configuration, it is easy to position the second window portion 20b within the range of the diameter of the distal end surface of the endoscope 40 in the radial direction F. As a result, the light of the optical fiber 100a leaking from the second window portion 20b is easily shielded by the distal end surface of the endoscope 40, and it is possible to suppress light irradiation to a portion other than the scheduled light irradiation region including the target site X through the second window portion 20b.

As described above, in the expanded state of the expansion portion 2a, the second window portion 20b is more inclined with respect to the central axis P than the first window portion 20a. Here, "the second window portion 20b is inclined with respect to the central axis P" means that a line L1 connecting two end portions (in the case of the embodiment illustrated in Fig. 4, the distal end 50b and the proximal end 50a) of a continuous line (hereinafter, the line is referred to as a "continuous line") formed by the surface of the second window portion 20b is inclined with respect to the central axis P in a cross section including the central axis P and parallel to the central axis P at an arbitrary position in the circumferential direction E passing through the second window portion 20b (see Fig. 4). In a case where the second window portion 20b includes the boundary between the expansion portion 2a and the proximal protrusion 2b1, the position of one end portion may be set as the boundary position between the expansion portion 2a and the proximal protrusion 2b1.

As described above, as a result of the second window portion 20b being inclined with respect to the central axis P, it is easy to visually recognize the distal side of the second window portion 20b through the second window portion 20b using the endoscope 40. Specifically, the endoscope 40 attached to the light irradiation device 1 is often arranged so that the surface of the lens 40b perpendicularly intersects the central axis P. Thus, as a result of the second window portion 20b being inclined with respect to the central axis P, the second window portion 20b and the lens 40b of the endoscope 40 easily face each other. As a result, it is easy to visually recognize the distal side of the second window portion 20b through the second window portion 20b using the endoscope 40. Conversely, in a case where the second window portion 20b is not inclined with respect to the central axis P, the second window portion 20b and the lens 40b of the endoscope 40 do not face each other, and it is difficult to visually recognize the distal side of the second window portion 20b through the second window portion 20b.

As described above, in the expanded state of the expansion portion 2a, the second window portion 20b is more inclined with respect to the central axis P than the first window portion 20a. Here, "the second window portion 20b is more inclined with respect to the central axis P than the first window portion 20a" means that the inclination angle of the second window portion 20b is larger than the inclination angle of the first window portion 20a. The "inclination angle of the second window portion 20b" means a magnitude of an angle of a minor angle formed by the line L1 connecting the two ends (in the case of the embodiment illustrated in Fig. 4, the distal end 50b and the proximal end 50a) of the continuous line formed by the surface of the second window portion 20b and the central axis P in the cross section including the central axis P and parallel to the central axis P at an arbitrary position in the circumferential direction E passing through the second window portion 20b. For example, in a case where the line L1 is orthogonal to the central axis P, the inclination angle of the second window portion 20b is 90°. The line L1 of the present embodiment is inclined at an acute angle smaller than 90° with respect to the central axis P. In addition, the "inclination angle of the first window portion 20a" means a magnitude of an angle of a minor angle formed by a line L2 connecting two ends (in the case of the embodiment shown in Fig. 4, the distal end 60b and the proximal end 60a) of a continuous line formed by the surface of the first window portion 20a and the central axis P in a cross section including the central axis P and parallel to the central axis P at an arbitrary position in the circumferential direction E passing through the first window portion 20a. For example, in a case where the line L2 is parallel to the central axis P, the inclination angle of the first window portion 20a is 0°. The line L2 in the present embodiment is parallel to the central axis P (the inclination angle of the first window portion 20a is 0°).

As described above, as a result of the second window portion 20b being more inclined with respect to the central axis P than the first window portion 20a, the first window portion 20a can be easily visually recognized through the second window portion 20b as compared with a case where the second window portion 20b is not more inclined with respect to the central axis P than the first window portion 20a.

The first window portion 20a may be substantially parallel or parallel to the central axis P. Here, "the first window portion 20a is substantially parallel to the central axis P" means that the above-described "inclination angle of the first window portion 20a" is 5° or less. In addition, "the first window portion 20a is parallel to the central axis P" means that the above-described "inclination angle of the first window portion 20a" is 0°. As a result of the first window portion 20a being substantially parallel or parallel to the central axis P, the second window portion 20b can be easily more inclined with respect to the central axis P than the first window portion 20a, and the scheduled light irradiation region including the target site X located outside in the radial direction F of the balloon 2 can be easily irradiated with light.

In the expanded state, the expansion portion 2a of the present embodiment includes a cylindrical expansion main body portion 2a1 surrounding the central axis P, an expansion proximal portion 2a2 continuous with the proximal side of the expansion main body portion 2a1 and inclined toward the proximal side so as to approach the central axis P, and an expansion distal portion 2a3 continuous with the distal side of the expansion main body portion 2a1. The first window portion 20a of the present embodiment is formed in the expansion main body portion 2a1 of the expansion portion 2a, and the second window portion 20b is formed in the expansion proximal portion 2a2 of the expansion portion 2a.

In addition, the expansion proximal portion 2a2 of the present embodiment protrudes in a hemispherical shape from the expansion main body portion 2a1 to the proximal side in the expanded state of the expansion portion 2a. By forming the expansion proximal portion 2a2 in a hemispherical shape, the second window portion 20b disposed in the expansion proximal portion 2a2 also has a shape along the spherical surface. As a result of the second window portion 20b having a shape along the spherical surface, the lens 40b of the endoscope 40 can be more easily brought closer to the second window portion 20b than in a case where the second window portion 20b has a shape along the conical surface, for example. By bringing the lens 40b of the endoscope 40 closer to the second window portion 20b, the first window portion 20a can be easily visually recognized through the second window portion 20b. In particular, in a case where a liquid is accommodated in the balloon 2 as a fluid, a difference in refractive index of light is likely to occur between the inside of the balloon 2 and the space between the second window portion 20b and the lens 40b of the endoscope 40. Thus, light reflected in the living body is less likely to be focused on the lens 40b via the first window portion 20a and the second window portion 20b, and it may be difficult to visually recognize the first window portion 20a through the second window portion 20b. On the other hand, by forming the expansion proximal portion 2a2 of the balloon 2 in a hemispherical shape, when the lens 40b of the endoscope 40 is brought close to the second window portion 20b, a liquid (for example, a liquid for cleaning the lens 40b, a gel applied in advance before the balloon 2 is inserted into the endoscope 40, a body fluid, or the like) tends to be stored between the second window portion 20b and the lens 40b of the endoscope 40 due to surface tension. In other words, by forming the expansion proximal portion 2a2 of the balloon 2 in a hemispherical shape and storing the liquid between the second window portion 20b and the lens 40b of the endoscope 40, the difference in refractive index between the inside of the balloon 2 and the space between the second window portion 20b and the lens 40b of the endoscope 40 is reduced, and the first window portion 20a is more easily visually recognized through the second window portion 20b.

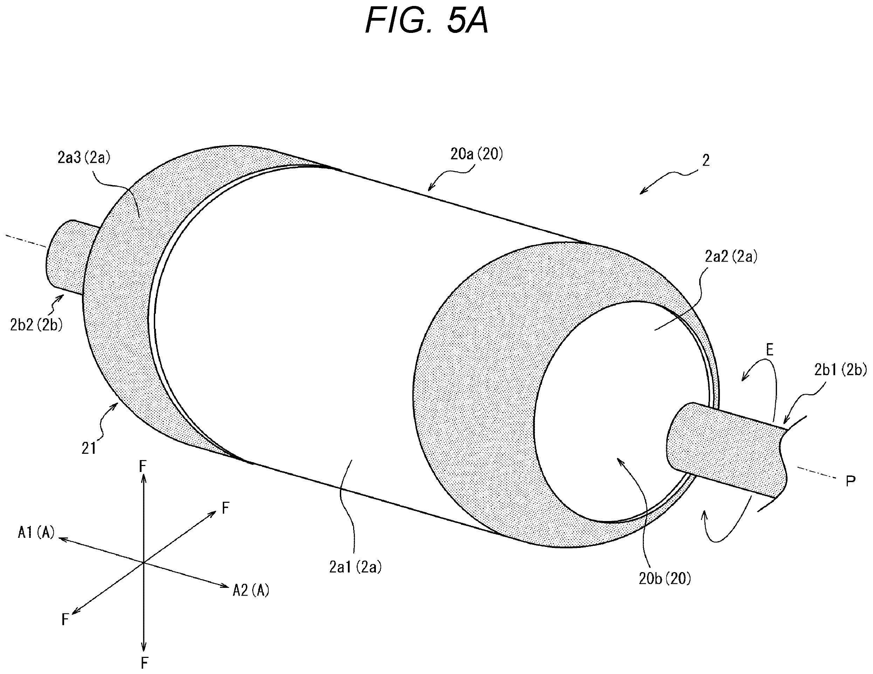

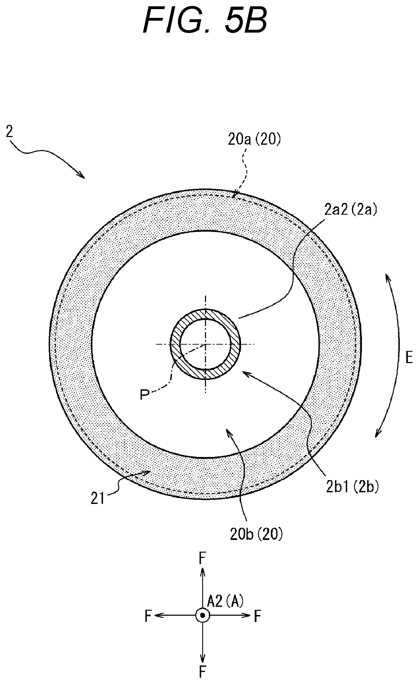

Fig. 5A is a view illustrating a modification of the balloon 2. More specifically, Fig. 5A is a perspective view illustrating an expanded state of the balloon 2 as a modification. Fig. 5B is a plan view of the balloon 2 illustrated in Fig. 5A as viewed from the proximal side in the axial direction A. In the modification illustrated in Figs. 5A and 5B, the second window portion 20b is disposed over the entire region in the circumferential direction E around the central axis P of the expansion portion 2a. With this configuration, even in a case where the lens 40b of the endoscope 40 is arranged at any position in the circumferential direction E with respect to the balloon 2, the first window portion 20a can be easily visually recognized through the second window portion 20b. In addition, light of a light disposed at the distal end portion (distal end portion) of the endoscope 40 passes through the first window portion 20a via the second window portion 20b and easily reaches the inside of the living body, and a brighter visual field is easily secured when the endoscope 40 is used.

Fig. 6A is a view illustrating another modification of the balloon 2. More specifically, Fig. 6A is a perspective view illustrating an expanded state of the balloon 2 as a modification. Fig. 6B is a plan view of the balloon 2 illustrated in Fig. 6A as viewed from the proximal side in the axial direction A. In the modification illustrated in Figs. 6A and 6B, the first window portion 20a is disposed over a limited angular range not over the entire circumference in the circumferential direction E around the central axis P of the expansion portion 2a. As a result, the light irradiation region by the light irradiation device 1 can be limited as compared with the case where the first window portion 20a is disposed over the entire circumference in the circumferential direction E (see Fig. 3A, and the like). The angle range of the first window portion 20a in the circumferential direction E of the present embodiment is 180 degrees. However, the angular range of the first window portion 20a in the circumferential direction E is not limited thereto, and can be appropriately changed.

In the modification illustrated in Figs. 6A and 6B, the second window portion 20b is arranged at a position different from the first window portion 20a in the circumferential direction E of the expansion portion 2a. By arranging the second window portion 20b in this manner, the first window portion 20a is more easily visually recognized through the second window portion 20b than in a case where the second window portion 20b is arranged at the same position as the first window portion 20a in the circumferential direction E. In the present modification, the entire second window portion 20b is arranged at a position different from the first window portion 20a in the circumferential direction E. However, if at least a portion of the second window portion 20b is arranged at a position different from the first window portion 20a in the circumferential direction E, a similar effect can be obtained.

The range of the second window portion 20b in the axial direction A is not particularly limited as long as it is arranged spaced apart on the proximal side of the first window portion 20a. The second window portion 20b may be formed, for example, in the entire region of the expansion proximal portion 2a2 of the expansion portion 2a.

The balloon, the light irradiation device, and the therapeutic method according to the present disclosure are not limited to the specific configurations and steps indicated in the above-described embodiments and modifications, and various modifications, changes, combinations, substitutions of steps, and the like, can be made without departing from the scope of the claims.

### Industrial Applicability

The present disclosure relates to a balloon, a light irradiation device, and a therapeutic method.

### Reference Signs List

1 Light irradiation device
1a Insertion portion
1b Operation portion
2 Balloon
2a Expansion portion
2a1 Expansion main body portion
2a2 Expansion proximal portion
2a3 Expansion distal portion
2b Protrusion
2b1 Proximal protrusion
2b2 Distal protrusion
5 Annular space
10 Transmissive layer
11 Light shielding layer
20 Window region
20a First window portion
20b Second window portion
21 Light shielding region
25 Tubular member
25a Inflation lumen
26 First tubular body
26a Accommodation space
27 Second tubular body
28 Hub
28a Port portion
28b Through hole
31 Closing member
32 Distal member
40 Endoscope
40a Main body portion
40a1 Through passage
40b Lens
50a Proximal end of continuous line formed by second window portion
50b Distal end of continuous line formed by second window portion
60a Proximal end of continuous line formed by first window portion
60b Distal end of continuous line formed by first window portion
80 Hollow organ
100 Light irradiation unit
100a Optical fiber
100a1 Light emission unit
200 Endoscope system
A Axial direction of light irradiation device
A1 Insertion direction
A2 Removal direction
B Circumferential direction of light irradiation device
C Radial direction of light irradiation device (example
of radial direction of tubular body)
E Circumferential direction of expansion portion of balloon
F Radial direction of expansion portion of balloon
L1 Line connecting proximal end and distal end of continuous line formed by second window portion
L2 Line connecting proximal end and distal end of continuous line formed by first window portion
∘ Central axis of light irradiation device
P Central axis of expansion portion of balloon
X Target site

## Claims

1. A balloon insertable into a living body,
the balloon comprising an expansion portion that defines a central axis extending along an insertion direction from a proximal side to a distal side,
the expansion portion comprising:
a transmissive layer capable of transmitting light emitted inside to an outside; and
a light shielding layer covering the transmissive layer and having a transmittance of the light lower than a transmittance of the transmissive layer,
the expansion portion comprising:
a window region in which the transmissive layer is not covered with the light shielding layer; and
a light shielding region in which the transmissive layer is covered with the light shielding layer, and
the window region of the expansion portion in an expanded state comprising:
a first window portion; and
a second window portion arranged spaced apart on the proximal side with respect to the first window portion and more inclined with respect to the central axis than the first window portion.

2. The balloon according to claim 1, wherein the first window portion is substantially parallel or parallel to the central axis.

3. The balloon according to claim 1 or 2, wherein the second window portion is disposed over an entire region in a circumferential direction around the central axis.

4. The balloon according to claim 1 or 2, wherein in the expansion portion, a distal side of the first window portion is comprised only of the light shielding region.

5. The balloon according to claim 1 or 2, wherein
the expansion portion in the expanded state comprises:
a cylindrical expansion main body portion surrounding the central axis; and
an expansion proximal portion continuous with a proximal side of the expansion main body portion and inclined toward the proximal side to approach the central axis,
the first window portion is arranged in the expansion main body portion, and
the second window portion is arranged in the expansion proximal portion.

6. The balloon according to claim 5, wherein the expansion proximal portion protrudes in a hemispherical shape from the expansion main body portion to the proximal side.

7. The balloon according to claim 1 or 2, wherein the first window portion is disposed over a limited angular range not over an entire circumference in the circumferential direction around the central axis of the expansion portion.

8. The balloon according to claim 7, wherein at least a portion of the second window portion is arranged at a position different from the first window portion in the circumferential direction.

9. A light irradiation device comprising:
the balloon according to claim 1 or 2; and
a tubular body that internally defines an accommodation space accommodating or capable of accommodating a light irradiation unit that emits the light,
wherein the balloon covers a circumference in a radial direction of the tubular body.

10. A therapeutic method using the balloon according to claim 1, the therapeutic method comprising:
a balloon position adjustment step of adjusting a position of the first window portion while visually recognizing the first window portion through the second window portion.

11. The therapeutic method according to claim 10, wherein in the balloon position adjustment step, the position of the first window portion is adjusted by moving the balloon in a direction parallel to the central axis.

12. The therapeutic method according to claim 10 or 11, wherein in the balloon position adjustment step, the position of the first window portion is adjusted by rotating the balloon in a circumferential direction around the central axis.

13. A therapeutic method using the balloon according to claim 1, the therapeutic method comprising:
an imaging device insertion step of inserting an imaging device into a living body;
a balloon insertion step of inserting the balloon into the living body through a through passage of the imaging device or in parallel with an outer surface of the imaging device;
an expansion step of expanding the balloon in the living body;
a lens position adjustment step of bringing a lens at a distal end of the imaging device close to the second window portion of the balloon;
a balloon position adjustment step of adjusting a position of the first window portion so that a scheduled light irradiation region in the living body and the position of the first window portion are aligned while visually recognizing the first window portion through the second window portion; and
a light irradiation step of irradiating an inside of the living body with the light in a state in which the scheduled light irradiation region and the position of the first window portion are aligned.

14. A therapeutic method using the balloon according to claim 1, the therapeutic method comprising:
an imaging device insertion step of inserting an imaging device into the living body;
a balloon insertion step of inserting the balloon into the living body through a through passage of the imaging device or in parallel with an outer surface of the imaging device;
a first expansion step of expanding the balloon in the living body while adjusting the balloon to a first expansion amount;
a lens position adjustment step of bringing a lens at a distal end of the imaging device close to the second window portion of the balloon; and
a balloon position adjustment step of adjusting a position of the first window portion of the balloon adjusted to the first expansion amount so that a scheduled light irradiation region in the living body and the position of the first window portion are aligned while visually recognizing the first window portion through the second window portion;
a second expansion step of expanding the balloon in the living body while adjusting the balloon to a second expansion amount larger than the first expansion amount after the balloon position adjustment step; and
a light irradiation step of irradiating an inside of the living body with the light in a state in which the scheduled light irradiation region and the first window portion are aligned after the second expansion step.
